# EUROPEAN PATENT SPECIFICATION

(11) **EP 3 112 680 B1**
(45) Date of publication and mention of the grant of the patent: **20.06.2018**
(21) Application number: 16183264.7
(22) Date of filing: 24.03.2005
(51) Int. Cl.: F04B 23/06

(54) **DUAL CYLINDER VACUUM PUMP FOR MEDICAL ASPIRATION SYSTEM**
DOPPELZYLINDERVAKUUMPUMPE FÜR EIN MEDIZINISCHES SAUGSYSTEM
POMPE À VIDE À DOUBLE CYLINDRE POUR SYSTÈME D'ASPIRATION MÉDICALE

(30) Priority: 26.03.2004 US 556963 P; 23.03.2005 US 88318
(43) Date of publication of application: 04.01.2017
(62) Divisional of application: 05729894.5
(73) Proprietor: Med-Logics, Inc., Athens, TX 75751 (US)
(72) Inventor: ROSS, Rod, LaRue, TX 75770 (US)
(74) Representative: Grünecker Patent- und Rechtsanwälte PartG mbB

(56) References cited:
- DE-C1- 19 727 623
- US-A- 4 543 044
- US-A1- 2002 151 835

## Description

### BACKC3ROtIND OF THE INVENTION

### 1. FIELD OF THE INVENTION

The present invention relates to a vacuum pump for a medical aspiration system.

### 2. BACKGROUND INFORMATION

Ophthalmic procedures are typically performed with instruments that have a tip located at the distal end of a handpiece. The handpiece is held by a surgeon who inserts the tip into the inner ocular chamber of an eye. By way of example, the surgeon may remove a cataracteous lens, or reattach a retina with the instrument. Document US 2002/151835 discloses a device used in the field of eye surgery. During a procedure, irrigation fluid is introduced into the eye to maintain the ocular pressure of the anterior chamber. The handpiece tip is coupled to an aspiration system that pulls the irrigation fluid and possibly tissue out of the anterior chamber. The tissue and irrigation fluid flow through an inner channel in the tip.

The aspiration system includes a pump coupled to an aspiration tube. The aspiration tube is connected to an outlet port of the handpiece. Most aspiration pumps are of the peristaltic type because the pump behavior is predictable. A peristaltic pump essentially pushes the air/fluid within the aspiration tube to create a vacuum pressure within the tube. The operation of a peristaltic pump creates surges in the pressure within the system. Pressure surges can be undesirable when performing delicate procedures such as retinal reattachment.

Some aspiration systems contain a venturi type pump. Venturi pumps do not creates pressure surges and are thus typically used in delicate ophthalmic procedures. Commercially available venturi pumps require a tank of compressed nitrogen gas. It is generally undesirable to have a pressurized gas tank in an operating environment. Additionally, venturi pumps are energy inefficient in creating a vacuum.

### SUMMARY OF THE INTENTION

A pump for a medical aspiration system. The pump includes a housing with an input port, an output port, a first cylinder and a second cylinder. A motor assembly of the pump moves a first plunger within the first cylinder and a second plunger within a second cylinder. The pump includes valve assemblies that control fluid communication between the input/output ports and the cylinders.

### BRIEF DESCRIPTION OF THE DRAWINGS

Figure 1 is a schematic of an embodiment of a medical system;
Figure 2 is an illustration of a dual cylinder pump of the medical system.

### DETAILED DESCRIPTION

Disclosed is a dual cylinder pump that is used to create a vacuum in an aspiration tube of a medical system. The pump includes a first plunger that moves within a first cylinder and a second plunger that moves within a second cylinder. Movement of the plungers is controlled by a motor assembly. The pump includes valves that control the flow of fluid into and out of the cylinders so that one cylinder is pulling fluid from the aspiration tube while the other cylinder is discharging fluid. In this manner the pump is continuously pulling vacuum, thereby preventing vacuum surges found in peristaltic pumps of the prior art. Additionally, the pump is relatively energy efficient and does not require a separate nitrogen tank as required by commercially available venturi pumps of the prior art. The pump can be constructed as a cartridge that can be removed and disposed.

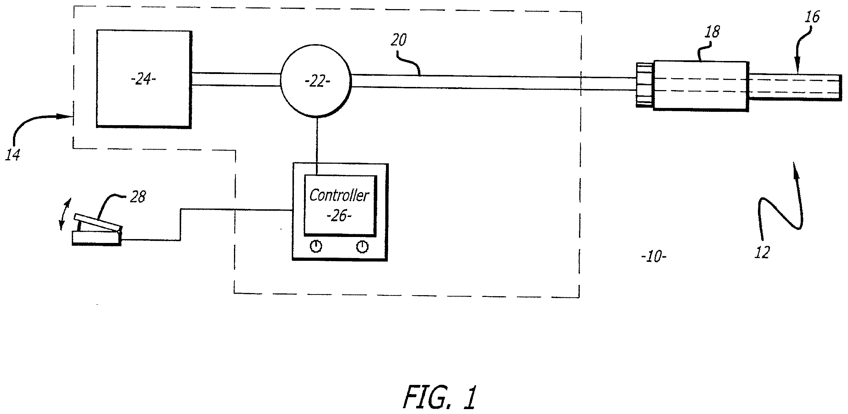

Referring to the drawings more particularly by reference numbers, Figure 1 shows an embodiment of a medical system 10 of the present invention. The system 10 may be used by a surgeon to perform ophthalmic medical procedures such as cataract lens removal, or retina reattachment.

The system 10 may include an instrument 12 that is coupled to an aspiration system 14. The instrument 12 may include a tip 16 that extends from a handpiece 18 and can be held by a surgeon. The tip 16 can be inserted into the eye of a patient.

The aspiration system 14 may include an aspiration tube(s) 20 that is coupled to the instrument 12. The aspiration tube 20 is connected to a vacuum pump 22 and a collection canister 24. The vacuum pump 22 creates a vacuum pressure within the aspiration tube 20 and a flow of fluid from the instrument 12 to the canister 24. The aspiration system 14 can pull emulsified tissue and fluid from the instrument 12 and into the canister 29.

The system 10 may include a controller 26 that is connected to the instrument 12 and the vacuum pump 22. The system 10 may further have a control device such as a foot pedal 28 that is connected to the controller 26. The surgeon can control the instrument 12 and/or the pump 22 thru the foot pedal 28. The controller 26 may include a processor, memory, etc. (not shown) that can operate the pump 22 in synchronization with the instrument 12. Although the foot pedal 28 is shown as being connected to the controller 26, the foot pedal 28 may be connected directly to the pump 22 and/or instrument 12.

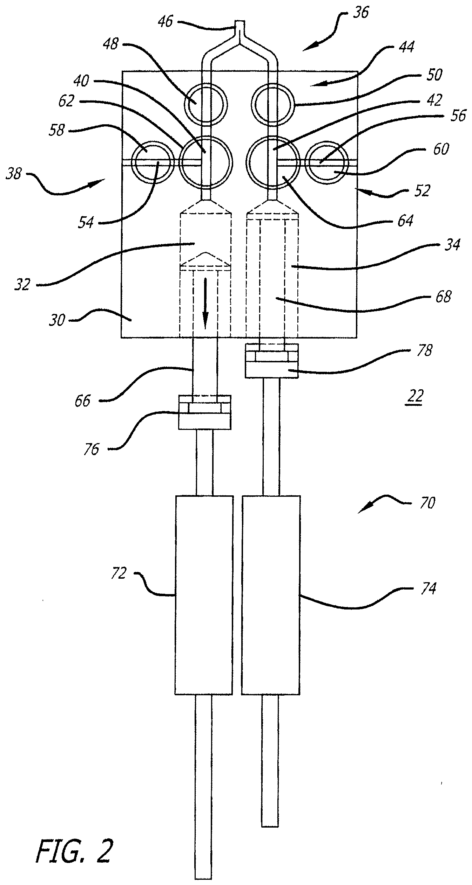

Figure 2 shows an embodiment of the vacuum pump 22. The pump 22 may include a housing 30 that has a first cylinder 32 and a second cylinder 34. The housing 30 also includes an input port 36 and an output port 38. The input port 36 includes channels 40 and 42 in fluid communication with the first 32 and second cylinders 34, respectively.

The pump 22 may have a first valve assembly 44 that controls the flow of fluid between the cylinders 32 and 34 and a manifold tube 46. The manifold tube 46 is connected to the aspiration tube 20 of the aspiration system 14. The first valve assembly 44 may include a first input valve 48 that controls the flow of fluid into the first cylinder 32 and a second input valve 50 that controls the flow of fluid into the second cylinder 34.

The pump 22 may further have a second valve assembly 52 that controls the flow of fluid between the cylinders 32 and 34 and outlet lines 54 and 56 of the output port. The second valve assembly 52 may include a first output valve 58 that controls the flow of fluid from the first cylinder 32 and a second output valve 60 that controls the flow of fluid from the second cylinder 34. The valves 48, 50, 58 and 60 may be controlled actuators or motors that are connected to and controlled by the controller 26 shown in Fig. 1.

The pump 22 may include pressure transducers 62 and 64 that sense the pressure within the first 32 and second 34 cylinders, respectively. The pressure transducers 62 and 64 can be connected to the controller 26 shown in Fig. 1, and provide pressure feedback information that can be used in a feedback control loop of the pump 22.

The pump 22 includes a first plunger 66 that moves within the first cylinder 32 and a second plunger 68 that moves within the second cylinder 34. The plungers 66 and 68 are moved by a motor assembly 70. The motor assembly 70 may include a first motor 72 that moves the first plunger 66 and a second motor 74 that moves the second plunger 68. The motors 72 and 74 may move the plungers 66 and 68 out of phase relative to each other. By way of example, the plungers 66 and 68 may move 180 degrees out of phase relative to each other. The motors 72 and 74 may be connected to the controller 26 which controls the timing and phase of the plungers 66 and 68. Although two motors are shown and described, it is to be understood that the plungers could be coupled to a single motor.

The motors 72 and 74 may be attached to the plungers 66 and 68 by couplers 76 and 78. The couplers 76 and 68 may be of the quick disconnect type so that the plungers 66 and 68, and housing 30 can be detached from the motor assembly 70. This allows the housing 30 and plungers 66 and 68 to be packaged as a cartridge that can be detached after a medical procedure. The plungers 66 and 68 may be of the syringe type that can be readily discarded and replaced. The housing 30 can be sterilized for reuse in the system 10. The valve actuators and pressure transducers may also be attached to the housing 30 in a sealed and readily detachable manner so that these components do not have to be sterilized after each procedure.

The controller 26 may control the motors 72 and 74 and the valve assemblies 44 and 52 in the following manner. The first input valve 48 may be opened to provide fluid communication between the manifold tube 46 and the first cylinder 32. The second input valve 50 is closed. The first motor 72 may pull the first plunger 66 in a direction indicated by the arrow. Movement of the plunger 66 pulls fluid into the first cylinder 32.

When the first plunger 66 reaches an end of travel the first input valve 48 is closed and the second input valve 50 is opened. The second motor 74 then pulls the second plunger 68 to draw fluid into the second cylinder 34. During this second plunger movement the first motor 72 pushes the first plunger 66. The first output valve 58 is opened so that the fluid within the first cylinder 32 is pushed out of the pump 22. The motors and valves are operated so that one of the cylinders is pulling in fluid while the other cylinder is pushing out fluid. In this manner a continuous vacuum is created in the aspiration tube. There are not sudden surges as found in prior art peristaltic pumps.

To maintain a continuous vacuum level, the second plunger 68 may begin to pull a vacuum in cylinder 34 as the first plunger 66 nears the end of travel in cylinder 32. Valve 50 may be closed during movement of the second plunger until the transducers 62 and 64 sense the same pressure wherein valve 50 is opened and valve 48 is closed.

While certain exemplary embodiments have been described and shown in the accompanying drawings, it is to be understood that such embodiments are merely illustrative of and not restrictive on the broad invention, and that this invention not be limited to the specific constructions and arrangements shown and described, since various other modifications may occur to those ordinarily skilled in the art.

For example, although multiple values 48, 50, 58 and 60 are shown and described, it is to be understood that the pump 22 may have other value arrangements. By way of example the pump 22 may have a single four-way valve.

Further preferred examples of the invention are as follows:
1. A pump for a medical aspiration system, comprising:
   a housing that has an input port, an output port, a first cylinder and a second cylinder;
   a first plunger that moves within said first cylinder;
   a second plunger that moves within said second cylinder;
   a motor assembly that moves said first and second plungers within said first and second cylinders, respectively; and,
   a valve assembly that controls fluid communication between said first and second cylinders, and said input and output ports.
2. The pump of example 1, wherein said motor assembly includes a first motor connected to said first plunger by a first coupler and a second motor connected to said second plunger by a second coupler.
3. The pump of example 1, wherein said first and second plungers move out of phase relative to each other.
4. The pump of example 1, wherein said valve assembly includes a first input valve that controls fluid communication between said input port and said first cylinder and a second input valve that controls fluid communication between input port and said second cylinder.
5. The pump of example 1, wherein said valve assembly includes a first output valve that controls fluid communication between said first cylinder and said output port and a second output valve that controls fluid communication between said second cylinder and said output port.
6. A medical system, comprising:
   an instrument;
   an aspiration tube coupled to said handpiece;
   a pump coupled to said aspiration tube, said pump including;
      a housing that has an input port coupled to said aspiration tube, an output port coupled to said aspiration tube, a first cylinder and a second cylinder;
      a first plunger that moves within said first cylinder;
      a second plunger that moves within said second cylinder ;
      a motor assembly that moves said first and second plungers within said first and second cylinders, respectively;
      a valve assembly that controls fluid communication between said first and second cylinders, and said input and output ports; and,
   a control device coupled to said pump.
7. The system of example 6, wherein said motor assembly includes a first motor connected to said first plunger by a first coupler and a second motor connected to said second plunger by a second coupler.
8. The system of example 6, wherein said first and second plungers move out of phase relative to each other.
9. The system of example 6, wherein said valve assembly includes a first input valve that controls fluid communication between said input port and said first cylinder and a second input valve that controls fluid communication between input port and said second cylinder.
10. The system of example 6, wherein said valve assembly includes a first output valve that controls fluid communication between said first cylinder and said output port and a second output valve that controls fluid communication between said second cylinder and said output port.
11. The system of example 6, wherein said control device includes a foot pedal.
12. The system of example 11, further comprising a controller coupled to said foot pedal and said pump.
13. A method for creating a vacuum pressure within an aspiration tube of a medical system, comprising:
   moving a first plunger within a first cylinder of a housing and moving a second plunger within a second cylinder of the housing; and,
   switching at least one valve to create fluid communication between an input port of the housing and the first and second cylinders and a fluid communication between an output port of the housing and the first and second cylinders, so that movement of the first and second plungers create a vacuum within an aspiration tube coupled to the input port.
14. The method of example 13, wherein the first and second plungers move out of phase with each other.
15. The method of example 13, wherein the first plunger is moved by a first motor and the second plunger is moved by a second motor.
16. The method of example 13, wherein the vacuum pressure irrigates fluid from an eye.
17. A pump cartridge that can be connected to an aspiration tube of a medical system and a motor assembly of a pump, comprising:
   a housing that has an input port that can be connected to the aspiration tube, an output port, a first cylinder and a second cylinder;
   a first plunger that moves within said first cylinder and can be connected to said motor assembly;
   a second plunger that moves within said second cylinder and can be connected to said motor assembly; and
   a valve assembly that controls fluid communication between said first and second cylinders, and said input and output ports.
18. The pump cartridge of example 17, wherein said first and second plungers move out of phase relative to each other.
19. The pump cartridge of example 17, wherein said valve assembly includes a first input valve that controls fluid communication between said input port and said first cylinder and a second input valve that controls fluid communication between input port and said second cylinder.
20. The pump cartridge of example 17, wherein said valve assembly includes a first output valve that controls fluid communication between said first cylinder and said output port and a second output valve that controls fluid communication between said second cylinder and said output port.

## Claims

1. A vacuum pump for creating vacuum in a medical aspiration system, the vacuum pump comprising:
a housing (30) comprising a first input port (36), a second input port (36), a first output port (38), a second output port (38), a first cylinder (32) communicating with the first input port (36) and the first output port (38), and a second cylinder (34) communicating with the second input port (36) and the second output port (36);
a first plunger (66) that moves within said first cylinder (32) alternately between a suction stroke and a discharge stroke;
a second plunger (68) that moves within said second cylinder (34) alternately between a suction stroke and a discharge stroke;
a motor assembly (70) that moves the first and second plungers (66, 68) within said first and second cylinders (32, 34), respectively;
a first actuated input valve (48) configured for controlling fluid flow into the first cylinder (32) from the first input port (36);
a second actuated input valve (44) configured for controlling fluid flow into the second cylinder (34) from the second input port (36);
a first actuated output valve (58) configured for controlling fluid flow from the first cylinder (32) to the first output port (38);
a second actuated output valve (56) configured for controlling fluid flow from the second cylinder (34) to the second output port (38);
a first pressure transducer (62) communicating with the first cylinder (32) and
configured for sensing a vacuum pressure therein;
a second pressure transducer (64) communicating with the second cylinder (34) and configured for sensing a vacuum pressure therein; and
an electronic control device (26) communicating with the first input valve (48), the second input valve (44), the first output valve (58), the second output valve (58), the motor assembly (70), the first pressure transducer (62) and the second pressure transducer (64), wherein the electronic controller (26) is configured for:
(a) alternating the suction stroke and the discharge stroke of the first plunger (66) with the suction stroke and the discharge stroke of the second plunger (58), respectively;
(b) controlling respective fluid flows into and out from the first cylinder (32) and into and out from the second cylinder (34), by actively controlling respective valve positions of the first input valve (48), the second input valve (44), the first output valve (58), and the second output valve (55);
(c) adjusting a user-set vacuum level at the first and second input ports (48, 44) in response to a vacuum level setting inputted by a user, and maintaining the user-set vacuum level, by monitoring pressure feedback information received from the first pressure transducer (62) and the second pressure transducer (64) and, based on the pressure feedback information, controlling respective movement speeds and directions of the first plunger (66) and the second plunger (68) and controlling respective positions of the first input valve (48), the first output valve (58), the second input valve (44) and the second output valve (56); and
(d) maintaining the user-set vacuum level during a transition from the suction stroke of the first plunger (66) to the suction stroke of the second plunger (68), by synchronizing respective positions of the first plunger (66), the second plunger (68), the first input valve (48), the first output valve (58), the second input valve (44) and the second output valve (66), such that initiation of the suction stroke of the second plunger (68) during the transition is delayed until a vacuum pressure in the second cylinder (34) is equal to the vacuum pressure in the first cylinder (32).

2. The vacuum pump of claim 1, wherein said motor assembly (70) includes a first motor (72) removably connected to said first plunger (66) by a first coupler (76) and a second motor (74) removably connected to said second plunger (68) by a second coupler (78).

3. The vacuum pump of claim 1, wherein said first and second plungers (66, 68) are movable out of phase relative to each other.

## Patentansprüche

1. Vakuumpumpe zum Erzeugen von Vakuum in einem medizinischen Aspirationssystem, wobei die Vakuumpumpe umfasst:
ein Gehäuse (30), das einen ersten Eingangsanschluss (36), einen zweiten Eingangsanschluss (36), einen ersten Ausgangsanschluss (38), einen zweiten Ausgangsanschluss (38), einen ersten Zylinder (32) der mit dem ersten Eingangsanschluss (36) und dem ersten Ausgangsanschluss (38) in Verbindung steht, sowie einen zweiten Zylinder umfasst, der mit dem zweiten Eingangsanschluss (36) und dem zweiten Ausgangsanschluss (36) in Verbindung steht;
einen ersten Kolben (66), der sich in dem ersten Zylinder (32) abwechselnd zwischen einem Ansaug-Hub und einem Ausstoß-Hub bewegt;
einen zweiten Kolben (68), der sich in dem zweiten Zylinder (34) abwechselnd zwischen einem Ansaug-Hub und einem Ausstoß-Hub bewegt;
eine Motor-Baugruppe (70), die den ersten und den zweiten Kolben (66, 68) in dem ersten bzw. dem zweiten Zylinder (32, 34) bewegt;
ein erstes betätigtes Eingangsventil (48), das so eingerichtet ist, dass es Fluidstrom in den ersten Zylinder (32) hinein über den ersten Eingangsanschluss (36) steuert;
ein zweites betätigtes Eingangsventil (44), das so eingerichtet ist, dass es Fluidstrom in den zweiten Zylinder (34) hinein über den zweiten Eingangsanschluss (36) steuert;
ein erstes betätigtes Ausgangsventil (58), das so eingerichtet ist, dass es Fluidstrom aus dem ersten Zylinder (32) zu dem ersten Ausgangsanschluss (38) steuert;
ein zweites betätigtes Ausgangsventil (56), das so eingerichtet ist, dass es Fluidstrom aus dem zweiten Zylinder (34) zu dem zweiten Ausgangsanschluss (38) steuert;
einen ersten Druckwandler (62), der mit dem ersten Zylinder (32) in Verbindung steht und so eingerichtet ist, dass er einen Vakuumdruck darin erfasst;
einen zweiten Druckwandler (64), der mit dem zweiten Zylinder (34) in Verbindung steht und so eingerichtet ist, dass er einen Vakuumdruck darin erfasst; sowie
eine elektronische Steuerungsvorrichtung (26), die mit dem ersten Eingangsventil (48), dem zweiten Eingangsventil (44), dem ersten Ausgangsventil (58), dem zweiten Ausgangsventil (58), der Motor-Baugruppe (70), dem ersten Druckwandler (62) und dem zweiten Druckwandler (64) in Verbindung steht, wobei die elektronische Steuerungsvorrichtung (26) so eingerichtet ist, dass sie:
a) jeweils zwischen dem Ansaug-Hub und dem Ausstoß-Hub des ersten Kolbens (66) und dem Ansaug-Hub und dem Ausstoß-Hub des zweiten Kolbens (58) wechselt;
b) jeweilige Fluidströme in den ersten Zylinder (32) hinein und aus ihm heraus sowie in den zweiten Zylinder (34) hinein und aus ihm heraus steuert, indem sie jeweilige Ventil-Positionen des ersten Eingangsventils (48), des zweiten Eingangsventils (44), des ersten Ausgangsventils (58) und des zweiten Ausgangsventils (55) aktiv steuert;
c) einen von einem Benutzer eingestellten Vakuum-Druckpegel an dem ersten und dem zweiten Eingangsanschluss (48, 44) in Reaktion auf eine von einem Benutzer eingegebene Vakuumpegel-Einstellung reguliert und den von dem Benutzer eingestellten Vakuumpegel aufrechterhält, indem sie von dem ersten Druckwandler (62) und dem zweiten Druckwandler (64) empfangene Druck-Rückkopplungsinformationen überwacht und auf Basis der Druck-Rückkopplungsinformationen jeweilige Bewegungs-Geschwindigkeiten und -Richtungen des ersten Kolbens (66) und des zweiten Kolbens (68) steuert und jeweilige Positionen des ersten Eingangsventils (48), des ersten Ausgangsventils (58), des zweiten Eingangsventils (44) sowie des zweiten Ausgangsventils (56) steuert; und
d) den von einem Benutzer eingestellten Vakuum-Druckpegel während eines Übergangs von dem Ansaug-Hub des ersten Kolbens (66) zu dem Ansaug-Hub des zweiten Kolbens (68) aufrechterhält, indem sie jeweilige Positionen des ersten Kolbens (66), des zweiten Kolbens (68), des ersten Eingangsventils (48), des ersten Ausgangsventils (58), des zweiten Eingangsventils (44) und des zweiten Ausgangsventils (66) so synchronisiert, dass Einleitung des Ansaug-Hubs des zweiten Kolbens (68) während des Übergangs verzögert wird, bis ein Vakuumdruck in dem zweiten Zylinder (34) dem Vakuumdruck in dem ersten Zylinder (32) gleich ist.

2. Vakuumpumpe nach Anspruch 1, wobei die Motor-Baugruppe (70) einen ersten Motor (72), der über eine erste Kupplung (76) lösbar mit dem ersten Kolben (66) verbunden ist, sowie einen zweiten Motor (74) enthält, der über eine zweite Kupplung (78) lösbar mit dem zweiten Kolben (68) verbunden ist.

3. Vakuumpumpe nach Anspruch 1, wobei der erste und der zweite Kolben (66, 68) phasenversetzt zueinander bewegt werden können.

## Revendications

1. Pompe à vide pour créer le vide dans un système d'aspiration médicale, la pompe à vide comprenant:
un boîtier (30) comprenant un premier port d'entrée (36), un second port d'entrée (36), un premier port de sortie (38), un second port de sortie (38), un premier cylindre (32) communiquant avec le premier port d'entrée (36) et le premier port de sortie (38), et un second cylindre (34) communiquant avec le second port d'entrée (36) et le second port de sortie (36);
un premier piston (66) qui se déplace à l'intérieur dudit premier cylindre (32) alternativement entre une course d'aspiration et une course de décharge;
un second piston (68) qui se déplace à l'intérieur dudit second cylindre (34) alternativement entre une course d'aspiration et une course de décharge;
un ensemble moteur (70) qui déplace les premier et second pistons (66, 68) à l'intérieur desdits premier et second cylindres (32, 34), respectivement;
une première soupape d'entrée actionnée (48) configurée pour commander l'écoulement de fluide dans le premier cylindre (32) à partir du premier port d'entrée (36);
une seconde soupape d'entrée actionnée (44) configurée pour commander l'écoulement de fluide dans le second cylindre (34) à partir du second port d'entrée (36);
une première soupape de sortie actionnée (58) configurée pour commander l'écoulement de fluide depuis le premier cylindre (32) vers le premier port de sortie (38);
une seconde soupape de sortie actionnée (56) configurée pour commander l'écoulement de fluide depuis le second cylindre (34) vers le second port d'entrée (38);
un premier transducteur de pression (62) communiquant avec le premier cylindre (32) et configuré pour détecter une pression de vide dans celui-ci;
un second transducteur de pression (64) communiquant avec le second cylindre (34) et configuré pour détecter une pression de vide dans celui-ci; et
un dispositif de commande électronique (26) communiquant avec la première soupape d'entrée (48), la seconde soupape d'entrée (44), la première soupape de sortie (58), la seconde soupape de sortie (58), l'ensemble moteur (70), le premier transducteur de pression (62) et le second transducteur de pression (64), dans lequel le dispositif de commande électronique (26) est configuré pour:
(a) faire alterner la course d'aspiration et de la course de décharge du premier piston (66) avec la course d'aspiration et la course de décharge du second piston (58), respectivement;
(b) commander les écoulements de fluide respectifs dans le premier cylindre (32) et hors de celui-ci, de même que le second cylindre (34) et hors de celui-ci, en commandant activement les positions de soupape respectives de la première soupape d'entrée (48), de la seconde soupape d'entrée (44), de la première soupape de sortie (58) et de la seconde soupape de sortie (55);
(c) ajuster un degré de vide défini par l'utilisateur au niveau des premier et second ports d'entrée (48, 44) en réponse à un réglage de degré de vide entré par un utilisateur, et maintenir le degré de vide défini par l'utilisateur, en surveillant les informations de retour de pression reçues du premier transducteur de pression (62) et du second transducteur de pression (64) et, en fonction des informations de retour de pression, en contrôlant les vitesses de mouvement et les directions respectives du premier piston (66) et du second piston (68) et en contrôlant les positions respectives de la première soupape d'entrée (48), de la première soupape de sortie (58), de la seconde soupape d'entrée (44) et de la seconde soupape de sortie (56); et
(d) maintenir le degré de vide défini par l'utilisateur pendant une transition de la course d'aspiration du premier piston (66) vers la course d'aspiration du second piston (68), en synchronisant les positions respectives du premier piston (66), du second piston (68), de la première soupape d'entrée (48), de la première soupape de sortie (58), de la seconde soupape d'entrée (44) et de la seconde soupape de sortie (66), de sorte que l'amorçage de la course d'aspiration du second piston (68) pendant la transition est retardé jusqu'à ce qu'une pression de vide dans le second cylindre (34) soit égale à la pression de vide dans le premier cylindre (32).

2. Pompe à vide selon la revendication 1, dans laquelle ledit ensemble moteur (70) comprend un premier moteur (72) relié de manière amovible audit premier piston (66) par un premier coupleur (76) et un second moteur (74) relié de façon amovible audit second piston (68) par un second coupleur (78).

3. Pompe à vide selon la revendication 1, dans laquelle lesdits premier et second pistons (66, 68) sont mobiles en déphasage l'un par rapport à l'autre.
